# EUROPEAN PATENT SPECIFICATION

(11) **EP 3 656 388 B1**
(45) Date of publication and mention of the grant of the patent: **24.12.2025**
(21) Application number: 20150599.7
(22) Date of filing: 25.11.2014
(51) Int. Cl.: A61K 35/64, A61K 35/644, A61K 31/375, A61K 31/723, A61K 33/00, A61K 36/61, A61K 38/48, A61K 8/04, A61K 8/19, A61K 8/66, A61K 8/67, A61K 8/73, A61K 8/98, A61P 17/00, A61P 17/02, A61K 36/185, A61K 9/06, A61K 9/00, A61K 47/02, A61K 33/10

(54) **COMPOSITION, SYSTEM AND USE FOR TREATING SKIN**
ZUSAMMENSETZUNG, SYSTEM UND VERWENDUNG ZUR BEHANDLUNG DER HAUT
COMPOSITION, SYSTÈME ET UTILISATION POUR LE TRAITEMENT DE LA PEAU

(30) Priority: 25.11.2013 US 201361908611 P
(43) Date of publication of application: 27.05.2020
(62) Divisional of application: 14863961.0
(73) Proprietor: D.T.R. Dermal Therapy Research Inc., London, ON N5X 2N9 (CA); Biomedical Concepts LLC, Gilbert, AZ 85234 (US)
(72) Inventor: PERLMUTTER, Alan Lorne, London, ON N5X 2N9 (CA); LEAMAN Jr., Donald H., Gilbert, AZ 85234 (US)
(74) Representative: Groth & Co. KB

(56) References cited:
- EP-B1- 1 677 811
- STEPHENS J M ET AL: "Phenolic compounds and methylglyoxal in some New Zealand manuka and kanuka honeys", FOOD CHEMISTRY, ELSEVIER LTD, NL, vol. 120, no. 1, 1 May 2010 (2010-05-01), pages 78 - 86, XP026799324, ISSN: 0308-8146, [retrieved on 20090926]
- RUSSELL K M ET AL: "Identification of Some Antibacterial Constituents of New Zealand Manuka Honey", JOURNAL OF AGRICULTURAL AND FOOD CHEMISTRY, AMERICAN CHEMICAL SOCIETY, BOOKS AND JOURNALS DIVISION, vol. 38, no. 1, 1 January 1990 (1990-01-01), pages 10 - 13, XP008149222, ISSN: 0021-8561, DOI: 10.1021/JF00091A002
- SASIKALA L ET AL: "Manuka honey loaded chitosan hydrogel films for wound dressing applications", INTERNATIONAL JOURNAL OF PHARMTECH RESEARCH 2013 SPHINX KNOWLEDGE HOUSE IND, vol. 5, no. 4, October 2013 (2013-10-01), pages 1774 - 1785, XP055366819, ISSN: 0974-4304
- ANONYMOUS: "Natural Skin Care: Exfoliating Honey Mask & Scrub", NATURE'S NURTURE, 20 October 2010 (2010-10-20), XP055345056, Retrieved from the Internet <URL:http://naturesnurtureblog.com/2012/10/29/natural-skin-care-exfoliating-honey-mask-scrub> [retrieved on 20170213]

## Description

### CROSS REFERENCE TO PRIOR APPLICATIONS

The present invention claims priority to US Application Number 61/908,611, filed on November 25, 2014.

### FIELD OF THE INVENTION

The present invention relates to a dressing for use in topical therapeutic treatment. More particularly, the invention relates to a dressing for use in desloughing, debriding, wound treatment and exfoliation.

### BACKGROUND OF THE INVENTION

Removal of non-viable body tissue is necessary or desirable for a number of reasons. Necrotic or non-viable tissue can develop within wound sites, including ulcerations in skin tissue, which may be due to chronic pressure and vascular insufficiency, and wounds due to physical trauma, burns (chemical or thermal), and sites of infection. Necrotic tissue inhibits the normal progression of healing by physically and biochemically obstructing the cascade of healing events in a wound site, and commonly harbors pathogens and their toxins. Removal of necrotic or non-viable skin tissue is a key step in healing such wounds. When healing medical devices such as Negative Pressure Wound Treatment ("NPWT") are indicated, it is important to thoroughly debride the wound of necrotic tissue and fibrotic tissue prior to using NPWT. This process is referred to as debridement. Debriding of the necrotic tissue is a first priority in healing the wound and is commonly accomplished by one or more of the following methods: (1) surgical debridement; (2) mechanical debridement; (3) enzymatic debridement; and (4) autolytic debridement. Surgical debridement and mechanical debridement, while effective, are often somewhat non-specific and can result in excessive tissue removal and pain to the patient.

Autolytic debridement involves the use of the body's own proteolytic enzymes (proteases) and moisture to rehydrate and digest necrotic tissue. It is typically only used in cases where the amount of necrotic tissue is relatively small and the slow rate of self-debridement is acceptable.

Enzymatic debridement, using proteolytic enzymes such as pepsin or papain/urea, is highly selective for non-viable tissue only. Examples of known enzymatic debridement methods and compositions include those taught in U.S. Pat. No. 6,548,556 and US Patent Application Publication No. 2009/0010910, as well as commercially known compositions such as Accuzyme^{®}. Although enzymatic debridement compositions and methods are known, there are a number of drawbacks and disadvantages to these compositions.

Adverse or harmful reactions have been reported in patients using topical enzymatic debriding compositions comprising papain as the active ingredient, including hypersensitivity reactions leading to hypotension (low blood pressure) and tachycardia (rapid heart rate). There is also a problem with possible allergic reactions. Papain is derived from papaya, and debriding compositions based on papain may also contain residual extracts from papaya. In addition, patients who are allergic to latex may exhibit cross-reactivity with papaya extracts. An alternative to papain is pepsin, a digestive enzyme. However, pepsin is most active in acidic environments (e.g. pH 2-3) such as the stomach, and is relatively inactive at pH 6.5 and higher. As a result, pepsin has very limited activity in the more neutral pH found in other body tissues such as skin and it is not very effective in debriding wound sites. In addition, commercially available pepsin is typically obtained from pig stomach, and is therefore not acceptable to some patients.

Another issue with topically applied enzymatic compositions is the ability of the composition to effectively coat and penetrate the wound site, and deliver the enzyme to the entirety of the wound site. In practice, this can be difficult, particularly if the wound site penetrates deeply into the body or encompasses multiple layers of different body tissues.

Removal of dead skin cells from the outermost layer (the stratum corneum) of the epidermis is desirable in certain cosmetic applications. Skin tissue is composed of two layers, the dermis and the epidermis. The dermis contains hair follicles, nerve endings, blood vessels, and oil and sweat glands. The epidermis is the outermost layer and unlike the dermis, it does not contain blood vessels. The epidermis is composed of a number of different layers and within the innermost (basal) layers of the epidermis are actively dividing skin cells known as keratinocytes. With each round of cell division, a generation of cells is pushed to the next level upwards, until they finally reach the outermost layer of the epidermis, the stratum corneum. The stratum corneum is composed of a thin layer of flattened, dead skin cells filled with keratin, and bound together by lipids. The outermost layer of dead skin cells of the stratum corneum sheds off naturally, and is continually replaced by newer layers below. On average, it takes about 28 days for a newly divided daughter cell to migrate from the basal layer of the epidermis to the outermost layer of the stratum corneum, where it can be shed off; this process is known as cell turnover. However, the rate of cell turnover can be affected by a number of factors, including hormonal fluctuations, increasing age, poor health and the local environment (e.g. dry weather). A build-up of dead skin cells in the outermost layer of skin can cause the skin to appear dull, dry and ashy. In addition, a build-up of dead skin cells and sebum may plug pores, providing an ideal site for bacterial infection, which then leads to acne breakouts. Superficial exfoliation of the outermost layer of skin to remove dead skin cells is thus highly useful for cosmetically improving skin appearance, and it is also useful for overcoming active acne and preventing future acne breakouts.

Exfoliation of skin tissue can be carried out by mechanical means or by chemical means. Examples of mechanical methods of exfoliation include hand-held tools such as scrubbing brushes, cloths and sponges, as well as cleansing compositions containing small beads or granules that act as a gentle abrasive when applied to the skin. However, there are risks associated with mechanical methods of exfoliation: users may scrub too hard or too often, and cause damage to the skin. This may result in irritation or drying out of the skin, which can lead to further problems with the skin.

The most common examples of chemical-based methods of cosmetic exfoliation include organic acids and retinoids. Weak organic acids, including beta hydroxy acids (e.g. salicylic acid) and alpha hydroxy acids, work as keratolytic agents, loosening and softening the outermost layer of the epidermis and thus helping to slough off dead skin cells. Alpha hydroxy acids, sometimes referred to as fruit acids, include glycolic acid, lactic acid, citric acid, mandelic acid and malic acid. Low concentrations of alpha and/or beta hydroxy acids are available in a variety of over-the-counter (OTC) cosmetic products, such as masks, creams, cleansers, lotions and toners. Application of such acids can cause stinging, redness and irritation, particularly those individuals with sensitive skin. Stronger organic acids, such as phenol and trichloroacetic acid, and higher concentrations of glycolic acid (e.g. >10%), penetrate the skin more deeply and can cause chemical burns. As such, these acids are not available for over-the-counter use and may only be applied by experienced medical professionals. Retinoids are a class of chemical compounds that trigger an increase in the rate of cell turnover. The use of retinoids is not without risk: there are numerous side effects associated with retinoid use, include sun sensitivity, redness, irritation, and peeling, flaking skin. A number of retinoid compounds are only available through prescription by a doctor. As well, retinoids, particularly oral forms, are contraindicated during suspected or confirmed pregnancy, due to the known association of retinoids with defects in fetal development. In addition to the above, benzoyl peroxide is commonly used for acne treatment, as it is both antimicrobial and an exfoliant. However, it often causes skin irritation and dryness.

Exfoliation of the epidermis with concomitant moisturization is necessary to manage certain chronic skin conditions such as ichthyosis, psoriasis, seborrheic dermatitis and eczema. Current treatments for these skin conditions typically include corticosteroids, coal tar, occlusive ointments and creams. In certain cases of icthyosis and psoriasis, retinoids may be prescribed. However, coal tar is often a treatment of last resort, due to the unpleasant smell and the unknown risks of long-term, low level exposure to potential carcinogens found in coal tar. Corticosteroids cannot be used for long periods of time, as it can cause permanent thinning of the skin and telangiectasia.

Honey has historically been used to treat a wide variety of medical concerns, including wounds, gastrointestinal disorders, allergies and infections of the upper respiratory tract. It is known to promote the healing of wounds as it provides numerous beneficial effects, including being anti-inflammatory, antioxidant, antimicrobial and anti-bacterial.

Honey is composed primarily of sugars, mainly fructose and glucose. Most types of honey are acidic due to the presence of naturally occurring organic acids, and have an average pH of around 3.9, but can range anywhere from pH 3.2 to 6.1. The water content in honey is very low, with an average water activity (aw) of 0.6; the water that is present within honey is bound to the sugar molecules that make up the bulk of honey, thus making honey inhospitable to microbial growth. The antimicrobial effect of honey is mainly attributed to the following factors: its low water content, hydrogen peroxide (H2O2), methylglyoxal, the antimicrobial peptide bee defensin-1, and its acidic pH.

Honey facilitates wound healing through its strong osmotic action on body tissue: as honey contains very little moisture (i.e. has a low water activity), it draws wound exudate by osmosis from the surrounding tissue. By drawing exudate out of the wound site, there is a constant supply of proteases brought to the wound site, particularly at the interface between necrotic tissue and viable tissue. As a result, autolytic debridement or desloughing of non-viable body tissue can be significantly enhanced with the use of honey as a wound dressing. In addition, the absorption of wound exudate by honey inhibits colonization of the wound site with microbial growth.

Honey also enhances wound healing by inhibiting microbial growth within the wound site, and inhibiting oxidative reactions that cause oxidative stress and inflammation. In most types of honey, the acidic pH, low water content and enzymatic production of hydrogen peroxide are the main factors contributing to the antimicrobial activity. However, for any given type of honey, the factors contributing to the antimicrobial activity and the overall level of antimicrobial activity are variable and highly dependent on the plant species that the honey bees originally fed upon. Honey derived from bees that have fed exclusively on Leptospermum scoparium, commonly referred to as Manuka honey, as well as honey derived from bees that have fed on other species of Leptospermum species, is known to have exceptional anti-microbial effect [1]. Manuka honey, as produced from Leptospermum species and particularly Leptospermum scoparium, contains significantly higher concentrations of methylglyoxal compared to other types of honey. The high concentration of methylglyoxal in Manuka honey is believed to be a major contributing factor to the observed antimicrobial effect [2]. Concentrations of methylglyoxal in Manuka honey can vary widely, dependent on the bees and the cultivar(s) of Leptospermum species that have been fed upon; however, a minimum methylglyoxal concentration of around 150 mg/kg is believed to be directly responsible for the observed antimicrobial effect [3]. The antimicrobial effect due to methylglyoxal in Manuka honey is a non-peroxide effect that is sometimes also referred to as the "Unique Manuka Factor" ("UMF"), a commercial rating scale that compares observed antimicrobial activity to solutions of phenol in water (e.g. a UMF of 10 is equivalent to the antimicrobial effect of 10% v/v phenol in water).

Although honey has numerous beneficial effects on wound treatment, it is typically in the form of a flowable liquid and can be difficult to keep in place, particularly when treating chronic wounds that may require long-term care. Specialized dressings may be required. For example, U.S. Patent No. 7,714,183 discloses a wound dressing impregnated with honey, as a means of keeping honey secure over the wound site. However, such dressings may actually inhibit wound healing by slowing down collagen deposition [4]. Also, as honey is typically acidic, the application of honey to a wound site may cause pain or discomfort to the patient.

There is evidence that carbon dioxide gas is beneficial for treating wound sites and promoting wound healing [5]. This is believed to be due to the Bohr effect, wherein hemoglobin's binding affinity to oxygen is inversely related to local acidity and carbon dioxide concentration. With an increase in local carbon dioxide concentration, the binding affinity of hemoglobin decreases. In addition, the body reacts by increasing blood flow to areas where local carbon dioxide concentration is increased. This may be observed as an increase in microcirculation of blood flow surrounding the wound area: this increase in circulation provides greater local oxygen availability and nutrient flow to the wound site, while also helping to removing wastes from the wound site. A carbon dioxide-enriched environment also minimizes the possibility of infection by aerobic bacteria. At the same time, there is no toxicity or major side effects to the patient to applying carbon dioxide. However, a disadvantage of carbon dioxide therapy is the need for specialized equipment in order to create a localized environment surrounding the wound site that can be filled with a sufficient concentration of carbon dioxide to observe an effect. This requires a source of carbon dioxide (usually in the form of a pressurized gas cylinder) and a method of delivering carbon dioxide to form a localized environment around the wound site, which may in turn require tubing and a syringe to inject carbon dioxide into the wound site and a physical method to enclose the wound site, e.g. adhesive dressings. As a result, carbon dioxide therapy is not a method that can be readily carried out without specialized equipment, and often requires the assistance of a skilled practitioner. An alternative, however, is provided in US 2006/0150988, which describes a cosmetic treatment method wherein carbon dioxide is transdermally delivered using a polymer solution.

There exists a need for methods and agents for removing non-viable or necrotic tissue from wound sites that overcomes at least one of the deficiencies known in the art. There also exists a need for compositions for exfoliation of the skin and/or to improve skin appearance, which overcomes at least one of the deficiencies known in the art.

### SUMMARY OF THE INVENTION

The invention provides a dressing for use in topical therapeutic treatment comprising a first layer and a second layer, whereby:
- the first layer comprises honey; and,
- the second layer comprises a pharmaceutically acceptable carbonate (CO3-2) and/or bicarbonate (HCO3-1) salt;
characterized in that the honey is in a dry, granular form and the carbonate and/or bicarbonate salt is in dry granular form.

The dressing can be used as a desloughing agent, a debriding agent, a wound treatment agent, a veterinary wound care agent, a chronic skin disorder treatment agent, a cosmetic skin exfoliant or any combination thereof.

Herein disclosed is also a composition comprising:
- honey;
- a pharmaceutically acceptable salt of carbonate (CO₃⁻²) or bicarbonate (HCO₃⁻¹), that is capable of reacting with an acid or an acidic solution to form carbon dioxide; and
wherein the pH of said composition is about 7.0 to 7.4.

The compositions described herein may be used as a desloughing agent, a debriding agent, a wound treatment agent, a veterinary wound care agent, a chronic skin disorder treatment agent, and a cosmetic skin exfoliant.

Disclosed herein is also a composition that comprises
- honey;
- about 2.5 to 20% w/w, preferably about 7.5 to 12.5% w/w, sodium bicarbonate;
- about 0.2 to 6% w/w, preferably about 2 to 5% w/w, xanthan gum; and
- about 0.2 to 10% w/w, preferably about 1 to 5% w/w, ascorbic acid.

### BRIEF DESCRIPTION OF THE DRAWINGS

The present invention will be further understood from the following detailed description of an embodiment of the invention, with reference to the drawings in which:
Figure 1 is a graph of observed desloughing in vitro of a sample of pig skin over time, with compositions of formulas (A) to (E) as denoted in Example 1; the concentrations of sodium bicarbonate in honey by percentage weight in each of formulas (A) to (E) are denoted as "HoneyMousse [concentration of NaHCO₃, % w/w]" in the legend;
Figure 2 is a graph of observed desloughing in vitro of a sample of pig skin over treatment time, comparing the desloughing efficiency of Formulation (E) (as described in Example 1), compared with honey alone (control), papain ointment and Santyl^{®} collagenase ointment;
Figure 3 is a series of photographs of observed changes in vivo, in a myiasis ulcer on a leg of a donkey, taken at Day 0, Day 7, Day 29 and Day 63, counting from the first day of treatment as described in Example 1(ii)(a);
Figure 4 is a series of photographs of observed changes in vivo, in an ankle wound on a donkey, taken at Day 0, Day 3 and Day 18, counting from the first day of treatment as described in Example 1(ii)(b);
Figure 5 is a series of photographs of observed changes in vivo, in a myiasis ulcer on the girth of a horse, taken at Day 0, Day 16, Day 21 and Day 32, counting from the first day of treatment as described in Example 1(ii)(d); and
Figure 6 is a series of photographs of observed changes in vivo, in a surgical excision wound of a human male patient (following excision of a keratoacanthoma), taken at Day 0, Day 6 and Day 7, counting from the first day of treatment as described in Example 1(ii)(c).

### DETAILED DESCRIPTION OF EMBODIMENTS

The terms "comprise", "comprises", "comprised" or "comprising" may be used in the present description. As used herein (including the specification and/or the claims), these terms are to be interpreted as specifying the presence of the stated features, integers, steps or components, but not as precluding the presence of one or more other features, integers, steps, components or a group thereof as would be apparent to persons having ordinary skill in the relevant art.

The term "about" as used herein with respect to concentrations, pH or other quantities is intended to indicate a variation of at least 10%.

As used herein (including the specification and/or the claims), the terms "debriding" and "debridement" refer to the removal of damaged, non-viable or necrotic tissue from a wound, and the term "slough" refers to the shedding of non-viable or necrotic tissue from living tissue. As used in herein, the terms "desloughing agent" and "debriding agent" refer to agents and compositions that remove, or aid in the removal of, non-viable or necrotic tissue from living tissue, by facilitating detachment of slough from living tissue within a wound site.

The present invention is based on the finding that a composition comprising honey and one or more pharmaceutically acceptable mineral salts of carbonate (CO₃⁻²) or bicarbonate (HCO₃⁻¹) (the salts capable of reacting with (1) moisture and an acid, or (2) an acidic solution, to form carbon dioxide), provides an unexpected significant enhancement of the removal of non-viable tissue, that is far greater than observed for either honey alone or carbon dioxide alone. The removal of non-viable tissue may be for the purposes as a desloughing, wherein the composition is used as a debriding agent, for the purposes of promoting wound healing, or it may be for superficial exfoliation of the epidermis to cosmetically enhance skin appearance. The enhancement of wound healing and therapeutic effect has been found to be far greater than would be expected from a simple additive effect of honey and carbon dioxide used separately to treat a wound site.

Honey contains naturally occurring organic acids, including gluconic acid, lactic acid, acetic acid, butyric acid, citric acid, malic acid, pyroglutamic acid and succinic acid. Gluconic acid is present in the highest concentration (up to 1% w/w), as it is produced by the oxidation of glucose by glucose oxidase, both of which are naturally occurring in honey. Medical grade Manuka honey typically has a pH in the range of pH 3.2 to 4.5. The concentration of total organic acids in Manuka honey from *Leptospermum scoparium* ranges from 0.17% w/w to 1.17% w/w. If desired, the pH of the honey may be further lowered by addition of any of the above-noted acids that naturally occur in honey, as well as other food grade or pharmaceutically acceptable weak organic acids such as ascorbic acid, and the alpha hydroxy acids, glycolic acid, citric acid and mandelic acid. In a preferred embodiment, gluconic acid and/or lactic acid, or salts thereof, may be added to the composition of the invention, as both of these acids and their salts provide beneficial effects. In the case of gluconic acid, it is capable of chelating metal ions, which may help to inhibit oxidative reactions that may otherwise damage healing tissue. In addition, both gluconic and lactic acids are humectants and thus provide moisturizing properties. In another preferred embodiment, the composition may further comprise ascorbic acid or a salt thereof. Ascorbic acid and its salts act as antioxidants and thus limit oxidate stress within the wound site, thereby promoting wound healing.

In a preferred embodiment, the honey used to prepare the composition is medical grade Manuka honey, with a minimum UMF rating of 10 (equivalent to antimicrobial effect of 10% phenol in water), a pH in the range of about 3.2 to 4.5, and a minimum methylglyoxal concentration of about 150 mg/kg. Preferably, the total organic acid content is in the range of about 0.17-1.17% w/w, with gluconic acid being the primary organic acid.

The addition of a pharmaceutically acceptable mineral salt of carbonate (CO₃⁻²) or bicarbonate (HCO₂⁻¹) to honey, initiates a reaction between the salt and the naturally occurring moisture and acids within the honey that evolves carbon dioxide gas within the honey. In a preferred embodiment, the composition according to an embodiment is in the form of a foam or mousse, wherein the foam comprises fine bubbles enriched in carbon dioxide. A slight molar excess of the mineral salt to the naturally occurring acids within the honey is added, such that the resultant composition has a pH that is preferably in the range of about 6.3-7.4.

The above-noted composition may be used as a desloughing composition, or debriding composition. The composition provides an elegant and simple method of covering a wound site with a carbon dioxide enriched atmosphere, without requiring any specialized equipment to introduce the carbon dioxide to the site of application. After applying, the portion of the composition exposed to air may dry to form a thin skin that further aids in sealing the carbon dioxide enriched atmosphere to the wound site. This also serves to seal the wound site from the surrounding environment, protecting it from contaminants. In the case where moist wound care is desired, the composition helps to help the wound site hydrated. The composition readily fills and penetrates the wound site, delivering both carbon dioxide and the honey to areas of the wound that would normally be difficult to access. As noted above, the honey helps to draw out wound exudate by its strong osmotic action on body tissue, and provides antimicrobial, anti-inflammatory and antioxidant effects.

The composition also provides a source for generating additional carbon dioxide: as the composition comes in contact with moisture from body tissue, or wound exudate, the excess mineral salt within the composition reacts with this moisture to form additional carbon dioxide.

The composition has an adherent contact surface that allows it to bind to both wet and dry wound surfaces, as well as wound dressings, which may be optionally applied over the composition. As the pH of the composition can be adjusted to about 6.3 to 7.4, the pH would be close to that of body tissues including epithelial and connective tissues, as well as interstitial fluid. Consequently, the application of the composition to the wound site is not expected to irritate or cause significant pain to the patient. The composition may be applied by an unskilled practitioner, or it may be self-applied by the patient.

The composition may be reapplied as required during the course of wound healing. It is expected that after the initial application, the composition will eventually lose activity over time as the honey will be diluted by moisture or wound exudate, the carbon dioxide comprised within the composition will gradually escape to the atmosphere, and/or the excess bicarbonate or carbonate salt will eventually be consumed.

In an embodiment, there is provided a system comprising the above-described composition, and an isotonic diluent buffer solution. The isotonic diluent solution may be used to dilute the composition to provide an irrigation solution that can be used to cleanse and rinse out the wound site before fresh composition is applied. This may be done before the initial application of composition is applied to the wound, or it may be done during the course of subsequent applications of the composition. In an embodiment, the diluent solution comprises ascorbic acid, sodium bicarbonate, and sodium chloride. Preferably, the diluent solution further comprises a surfactant and a pharmaceutically acceptable preservative. The diluent solution can be used to dilute the composition by any degree, as would be understood by persons skilled in the art. For example, in one embodiment, the diluent solution can be used to dilute the composition to a ratio (the volume of composition to the volume of diluent solution) of about 1:2 to 1:30. Any other dilution ratios within this range are also contemplated, such as 1:2, 1:5, 1:10, 1:15, 1:20, 1:25 and 1:30, inclusive of all ratios between these values. Various medical devices may be employed to apply the wound irrigation solution to the wound, including but not limited to: debriding sponges, pulsatile wound irrigation devices, mechanical spray irrigation devices (including spray and vacuum devices), squeeze bottle dispensers and the like.

Besides its use in promoting the healing of chronic wounds, the composition may also be used to promote the healing of other medical concerns, for example, oral ulcers, surgical wounds, trauma wounds, minor cuts, abrasions, burns, puncture wounds and sunburn.

In another embodiment, the composition may be used as a veterinary wound care agent, to promote wound healing in wild and domesticated animals. Such wounds may include lacerations, abrasions, myiasis (fly bite) ulcerations, and burns resulting from chemical, thermal or electrical exposure. For such veterinary uses, the composition may further comprise a suitable insect repellant such as citronella, in order to limit insect contact with the wound site.

It has also been discovered that the above-described composition is useful for a variety of cosmetic applications. For example, it may be used for superficial exfoliation of the epidermis to cosmetically improve skin appearance.

The composition would also be used to improve the appearance of hyperpigmented areas. This can be achieved by exfoliation to remove pigmented skin cells, revealing less pigmented skin below.

In cosmetics, sodium bicarbonate is sometimes used as a mechanical exfoliant in facial or body scrubs and it is also found in compositions that provide an antiseptic and deodorant effect; in food sciences, sodium bicarbonate or sodium carbonate (both in pure, solid form, or in aqueous solution) are sometimes used to break down protein, due to their basic pH. However, sodium bicarbonate can be overly harsh as a mechanical exfoliant and it can be irritating to the skin, and this effect would be common to any other mineral salts of carbonate or bicarbonate. As well, solutions of bicarbonate or carbonate salts are basic in pH and would also be irritating or damaging to the skin. The above-described composition is far superior to sodium bicarbonate alone, as it provides a mild yet effective means of superficial exfoliation of the epidermis without causing any irritation or damage to the skin. As an exfoliant, the composition is more effective than either honey alone, or mineral salts of carbonate or bicarbonate.

The composition may also be used to enhance recovery from acne, as it provides an antibacterial and anti-inflammatory effect, while at the same time, it exfoliates the epidermis and helps to clear infected pores. The composition may be applied as a mask that is left on the skin for a short period of time, and then rinsed off.

In comparison to other commonly used cosmetic exfoliants including mechanical exfoliants (e.g. cleansers containing small particles which act as abrasives; brushes, cloths and sponges), and chemical exfoliants such as alpha and beta hydroxy acids, retinoids, and topical acne treatments such as benzoyl peroxide, the above-noted composition is nonirritating and is well tolerated by most users, with low allergenic potential.

The composition may be also be used to manage and improve chronic skin conditions including ichthyosis, psoriasis, seborrheic dermatitis, atopic dermatitis and eczema. The composition forms an occlusive barrier on the skin, which provides moisturizing and humectant effects. The composition aids in exfoliation of the build-up of dead skin cells, a common feature in all of these skin conditions. At the same time, the composition serves to calm inflammation, inhibit secondary microbial infections, and promote healing of the skin.

In addition to the above, the composition may be provided in the form of a lip balm or lip ointment. As a lip balm, the composition enhances exfoliation of dried skin from the lips while moisturizing the lips. Both of these properties are highly useful to users in cold and/or dry climates. The composition also promotes the healing of cold sores and fever blisters, which are symptoms exhibited by patients suffering from other diseases or infections, such as Herpes Simplex I infection.

In an embodiment, the mineral salt is a pharmaceutically acceptable, water soluble salt, preferably a carbonate and/or bicarbonate salt, and wherein the salt evolves carbon dioxide (CO₂) gas upon contact with an acid and water. In a further embodiment, the one or more salts are selected from sodium bicarbonate, ammonium bicarbonate, potassium bicarbonate, calcium bicarbonate, sodium carbonate, potassium carbonate and combinations thereof. In a preferred embodiment, the salt is sodium bicarbonate.

In another embodiment, the honey is medical grade honey. In yet another embodiment, the honey is medical grade honey derived from bees that have fed primarily or exclusively on plants of *Leptospermum* sp. In a preferred embodiment, the honey is obtained from the hives of bees that have fed exclusively on *Leptospermum scoparium,* commonly referred to as Manuka, New Zealand tea tree, broom tea tree or simply tea tree. Honey of this source is also commonly referred to as Manuka honey.

In an embodiment, the composition is provided in the form of a foam or mousse, wherein said foam or mousse comprises bubbles or cells that further comprise carbon dioxide gas. The foam is preferably capable of generating additional carbon dioxide gas upon contact with acid and moisture. In another embodiment, the composition is provided as an ointment, cream, lotion, gel or balm, wherein said ointment, cream, lotion, gel, or balm is capable of generating carbon dioxide gas upon contact with acid and moisture. If the composition is provided in the form of an ointment, cream, lotion, gel or balm, it may further comprise bubbles or cells that further comprise carbon dioxide gas.

Thickening agents may be added to the composition to provide a thicker or stiffer consistency, which may be desirable under various circumstances. An example would be the care required for chronic wounds since, in such cases, the composition must stay in place for longer periods of time. Chronic wounds include but are not limited to ulcers, surgical wounds, and burns and those comprising necrotic tissue. Thickening agents may also help to absorb moisture such as wound exudate, thus preventing dilution of the composition, and extending the activity of the composition. Examples of suitable thickening agents include but are not limited to: polysaccharides such as starches, agar, alginic acid and salts thereof (e.g. sodium alginate), carrageenan, pectin, beta glucan, and natural gums such as xanthan gum, gum arabic, gum ghatti, guar gum, locust bean gum and combinations thereof. In a preferred embodiment, the thickening agent is xanthan gum. Other suitable thickening or gelling agents will be known to persons skilled in the art.

Suitable surfactants may also be added to the composition to aid in denaturation of non-viable tissue, and thus promote the debriding or desloughing effect. An example of such a surfactant is sodium lauryl sulfate. In an embodiment, the composition comprises about 0.0001 to 0.1% w/w sodium lauryl sulfate. Other suitable surfactants will be known to persons skilled in the art.

In another embodiment, the composition may further comprise one or more proteolytic enzymes (proteases). The protease provides enzymatic debridement to the composition and further enhances the activity of the composition for degradation and removal of non-viable or necrotic body tissue. Autolytic debridement, as brought on by application of honey, is considered to be slow acting, compared to enzymatic debridement. Thus, the addition of more or more proteases may extend the activity of the composition. The proteolytic enzyme may be plant-derived proteases, such as papain, actinidin, bromelain, ficain, or cucumisin, or bacteria-derived proteolytic enzymes. In a preferred embodiment, the composition comprises actinidin. In another preferred embodiment, the composition comprises a plant extract that comprises actinidin. The plant extract is preferably kiwi fruit extract, which contains actinidin. The plant proteases may be added to the composition in crude or purified form. If the composition comprises one or more plant-derived proteases, the composition may additionally contain antimicrobial agents such as methylglyoxal or its precursor, 1,3-dihydroxyacetone.

In yet another embodiment, the one or more proteolytic enzymes, if present, are preferably provided in their zymogen form. As used herein, the term "zymogen" will be understood to mean an inactive form of an enzyme that is converted to its active form by action of an activating agent. Such activating agent may include an acid, another enzyme or a combination thereof.

In order to maintain desired properties and to increase storage time (shelf life) over prolonged periods of time, the composition may be supplemented with one or more pharmaceutically acceptable preservatives as known in the art. Examples of preservatives that may be used in the present invention include methyl paraben, propyl paraben, butyl paraben, imidazolidinyl urea, tetradecyl-trimethyl ammonium bromide (Cetrimide^{®}), sodium benzoate, potassium sorbate, thymol, polyaminopropyl biguanide, and combinations thereof. Various other preservatives will be known to persons skilled in the art.

As described above, the composition comprises honey that is produced by bees that have fed mainly or exclusively on *Leptospermum* plant species. Preferably, the honey is Manuka honey produced from *Leptospermum scoparium.* In another embodiment, the Manuka honey has a pH of about 3.2 to 4.5 and a minimum methylglyoxal concentration of about 150 mg/kg. In one embodiment, particularly for wound treatment, the composition comprises about 80% (w/w) or more of honey.

In another embodiment, the composition is in the form of a foam or mousse, preferably comprising cells or bubbles of carbon dioxide. The composition may also be in the form of a gel, lotion, cream, ointment or balm. These other forms may further comprise cells or bubbles comprising carbon dioxide.

In yet another embodiment, the pharmaceutically acceptable salt is: sodium carbonate (Na₂CO₃), potassium carbonate (K₂CO₃), ammonium carbonate ((NH₄)₂CO₃), sodium bicarbonate (NaHCO₃), potassium bicarbonate (KHCO₃), ammonium bicarbonate ((NH₄)HCO₃) or any combination thereof. In one embodiment, the pharmaceutically acceptable salt is sodium bicarbonate (NaHCO₃).

The pharmaceutically acceptable salt may be present in the composition at about 2.5 to 20% w/w of the composition. In one aspect of the invention, the salt is present at about 5 to 15% w/w of the composition. In another aspect, the salt is present at about 7.5 to 12.5% w/w of the composition. In one aspect of the invention, the concentration of the salt may be varied according to the desired use of the composition. For example, if the composition is for use in treating a wound with necrotic tissue and malodor, the salt (e.g. sodium bicarbonate) concentration may be 12.5% -15.0%. If the composition is for use in treating a wound after all necrotic tissue has been removed and is in later stages of granulation, the salt concentration may be 7.5%-10%. If the composition is for use in treating a wound with completed granulation and where re-epithelialization has started, the salt concentration may be 2.5%-5.0%. Thus, as would be understood by persons skilled in the art, a higher salt concentration would be preferred when the composition is used in a wound at an early stage of healing. However, it will also be understood that such preferred embodiment of the invention is not intended to restrict the concentration of the salt component based on the state of the wound.

In an embodiment of the present invention, the composition may further comprise one or more organic acids. For example, the organic acids may be: gluconic acid, lactic acid, acetic acid, butyric acid, citric acid, malic acid, pyroglutamic acid, succinic acid, ascorbic acid, glycolic acid, lactic acid, citric acid, mandelic acid or any combination thereof. The one or more organic acids of the composition would be understood to be in addition to those acids naturally occurring in honey. In one embodiment, the organic acid used in the composition is ascorbic acid. The organic acids added to the composition may be present at a concentration of about 0.2 to 10% w/w of the composition. In one aspect, the acid is present at a concentration of about 1 to 5% w/w of the composition. Generally, the concentration of the organic acid would be that needed to adjust the pH of the composition from about 5 to about 7.4 and, preferably, from about pH 5.5 to about 6.5. More preferably, the pH of the composition is adjusted to be about 7 to 7.4. As would be understood, where the concentration of the carbonate and/or bicarbonate salt is high, resulting in a greater amount of CO₂ release, a higher concentration of the organic acid would be needed to adjust the pH of the composition to the aforementioned desired levels.

The composition may further comprise one or more thickening agents. For example, the thickening agents may be: polysaccharides, starches, agar, alginic acid and salts thereof, carrageenan, pectin, beta glucan, xanthan gum, gum arabic, gum ghatti, guar gum, locust bean gum, or any combination thereof. In one embodiment, the composition comprises xanthan gum. The thickening agents may be present at a concentration of about 0.2 to 6% w/w, with the concentration being dependent upon the desired viscosity of the composition. For example, a sufficient amount of thickening agent may be incorporated to provide a viscosity, at skin temperature, that is sufficient to cover and seal the site of application (e.g. the wound area) so as to provide an environment enriched in CO₂ and reduced in O₂. In one aspect, the thickening agents may be present at a concentration of about 1 to 5% w/w. In another aspect, the thickening agents may be present at a concentration of about 2 to 5% w/w.

In yet another embodiment, the composition may further comprise sodium lauryl sulfate. Sodium lauryl sulfate may present in the composition at a concentration of about 0.0001 to 0.1% w/w.

In another embodiment, the composition further comprises one or more proteolytic enzymes. For example, the proteolytic enzymes can be: papain, actinidin, bromelain, ficain, cucumisin, bacteria-derived proteolytic enzymes, or any combination thereof. The enzyme may be provided as an extract of the plant or bacteria that comprises one or more of the above-noted enzymes. In a further embodiment, the enzyme is actinidin. Actinidin may be provided in kiwi fruit or an extract thereof. Such an extract includes juice of the kiwi fruit, or a concentrate thereof.

In yet another embodiment, the composition further comprises one or more preservatives. For example the preservatives may be: methyl paraben, propyl paraben, butyl paraben, imidazolidinyl urea, tetradecyl-trimethyl ammonium bromide (Cetrimide^{®}), sodium benzoate, potassium sorbate, thymol, polyaminopropyl biguanide, or any combination thereof.

The composition may have an osmotic concentration at least 14.5 times the osmotic concentration of human blood.

In another aspect of the present invention, there is provided a use of the composition as described herein, as a desloughing agent or a debriding agent, a wound treatment agent, a veterinary wound care agent, and for the treatment of chronic skin disorders. Such skin disorders may comprise, for example: ichthyosis, psoriasis, seborrheic dermatitis, atopic dermatitis, and eczema. There is also provided a use of the composition as a cosmetic skin exfoliant.

In yet another aspect, there is provided a system of wound treatment comprising the composition according to any of the above-described embodiments and an isotonic diluent solution, preferably of about pH 7, comprising: about 0.015 M ascorbic acid; about 0.015 M sodium bicarbonate; and, about 0.12 sodium chloride;
- wherein the isotonic diluent solution is used to dilute said composition at a minimum ratio of 1 part said composition to 2 parts said diluent solution (1:2), thereby forming a wound irrigation solution; and wherein the wound irrigation solution is used to irrigate a site of treatment on a patient before application of said composition.

An advantage of the invention is that is provides an unexpected, and significant enhancement of the removal of non-viable tissue, that is far greater than observed for each of honey, carbon dioxide, and mineral salt of carbonate (CO₃⁻²) or bicarbonate (HCO₃⁻¹). The enhancement of wound healing and therapeutic effect is also far greater than would be expected from a simple additive effect of honey, carbon dioxide, and mineral salt of carbonate or bicarbonate, used separately to treat a wound site.

Another advantage of the invention is that it may be used as a desloughing or debriding agent, for the purposes of promotion of wound healing, or it may be for superficial exfoliation of the epidermis to cosmetically enhance skin appearance.

Yet another advantage of the invention is that it provides an elegant and simple method of covering a wound site with a carbon dioxide enriched atmosphere, and honey, both of which provide numerous beneficial effects in the promotion of wound healing, including but not limited to:
- providing a hyperosmotic environment (also referred to as an environment of negative osmotic pressure) that draws out and absorbs wound exudate;
- providing a means of stimulating oxygen and microcirculation to the wound site; and,
- providing a carbon dioxide-enriched micro-climate over the wound that discourages growth of aerobic bacteria.

Another advantage of the invention is that it is mild, with a pH that can be adjusted close to the pH of interstitial body fluids and body tissues including epithelial and connective tissues; consequently, the application of the composition to the wound site is not expected to irritate or cause significant pain to the patient.

Yet another advantage of the invention is that it is simple and does not require specialized equipment to use or apply: the composition may be applied by an unskilled practitioner, or it may be self-applied by the patient.

Another advantage of the invention is that it may be used on human patients or it may also be used to promote wound healing in wild or domesticated animals.

Another advantage of the invention is that it may be used for superficial exfoliation of the epidermis to cosmetically improve skin appearance. It may also be used to enhance recovery from acne, as it provides an antibacterial and anti-inflammatory effect, while at the same time, it exfoliates the epidermis and helps to clear infected pores. In addition, the invention may be also be used to manage and improve chronic skin conditions including ichthyosis, psoriasis, seborrheic dermatitis and eczema.

In all cases, an advantage of the invention is that it is much milder than known treatment methods, with low irritation and allergenic potential, while still providing effective treatment and management of the above-noted medical concerns.

Other and further advantages and features of the invention will be apparent to those skilled in the art from the following detailed description of an embodiment thereof, taken in conjunction with the accompanying drawings.

### Kits

In one aspect, there is provided a treatment kit comprising the honey component and the carbonate and/or bicarbonate salt component of the composition, as discussed above. Optionally, the kit may include any necessary instructions for forming and using the composition and/or any variety of containers or vials etc. for mixing the components to form the composition.

In one aspect, the honey component of the composition is provided in dry or powder form. Similarly, the carbonate and/or bicarbonate salt component may also be provided in dry or powder form. As would be understood, providing the components of the invention in dry form allows for extended shelf life. In the case of dried or powdered honey and dried or powdered carbonate/bicarbonate salt(s), the two components can be provided in a kit comprising one or two containers or packages for the honey, carbonate and/or bicarbonate salt, or a mixture thereof. In such case, the kit may further comprise a container or package containing a volume of water, which can be used to form the composition described above. As mentioned above, the kit may include a container into which the dry/powder components are combined with the water. In one example, the container may contain the necessary volume of water and the dry honey and salt components may be provided in a package or sachet. Alternatively, the container may be provided in an empty state, prepared to receive the dry/powder components and the required volume of water from an acceptable source.

In another aspect, the dried honey and carbonate/bicarbonate salt components can be combined into a dressing or other such application medium. Once wetted, the components of the composition would become wetted and thereby activated. In one aspect, the dressing would be adapted to form a foam or mousse as described above. The wetting of the dressing can be achieved by adding water or by using the natural exudate originating from a wound.

In one aspect, the above mentioned dressing may incorporate coated honey crystals, which are activated by the high pH of the bicarbonate once hydrated by added water or would excretions. Such coating may be provided by microencapsulation of the dried honey.

In another aspect, the dried honey, the carbonate/bicarbonate salt and the acid components can be combined together in dry form in a single airtight pouch. In one aspect, the honey may be coated (or microencapsulated) to allow for a release under certain conditions. In another aspect, neither of the ingredients would need to be coated as they would be inert in their dry state. The powder composition, such as the non-coated particles, can then be applied to a region to be treated, such as a skin ulcer, and then covered to allow the treatment phase to take place.

In the above description, the use of natural honey, in either liquid or dry (and/or encapsulated) form, has been discussed. However, in further aspects, such natural honey can be replaced with a synthetic honey or a honey substitute. For example, corn syrup of a suitable viscosity can be used instead of honey to entrap carbon dioxide released at the point of use due to the reaction of organic acid(s) and sodium bicarbonate. Similarly, suitable compositions of invert sugars such as corn syrup of such viscosity and low water content as to approximate honey (86% sugars) in a ratio of 55% fructose and 45% glucose may be used as alternatives to honey in necrotic wound and healing formulations as described above.

### Examples

Aspects of the invention will now be described with reference to various examples.

### Example 1: Preparation and Testing of Treatment Compositions

### (i) Preparation of Treatment Compositions

The following compositions were prepared. Medical grade Manuka honey derived from Leptospermum scoparium (Links Medical LLC) was used to prepare the compositions, wherein the medical grade honey had a pH 3.63, and was defined as having an activity equivalent to 16% phenol, also referred to as level 16 on the 20 point Unique Manuka Factor (UMF) scale. Either sodium bicarbonate or potassium bicarbonate could be used in the formulations noted below. The results shown below are for formulations prepared with sodium bicarbonate.

**Table 1: Compositions of honey and sodium bicarbonate (% w/w of total)**

| **Formulation** | **Honey (% w/w)** | **Sodium bicarbonate (NaHCO₃) (% w/w)** | **pH** | **As noted in** **Figure 1** |
|---|---|---|---|---|
| A | 97.5 | 2.5 | 6.35 | HoneyMousse 2.5% |
| B | 95.0 | 5.0 | 6.95 | HoneyMousse 5% |
| C | 92.5 | 7.5 | 7.0-7.2 | HoneyMousse 7.5% |
| D | 90.0 | 10.0 | 7.0-7.2 | HoneyMousse 10% |
| E | 87.5 | 12.5 | 7.2 | HoneyMousse 12.5% |
| Control | 100.0 | 0 | 3.63 | Honey Control |

Upon addition of sodium bicarbonate to honey, a composition in the form of a stable foam was formed, comprising minute cells, each cell enclosing a carbon-dioxide enriched atmosphere.

### (ii) In Vitro Testing of Debriding/Desloughing Efficiency

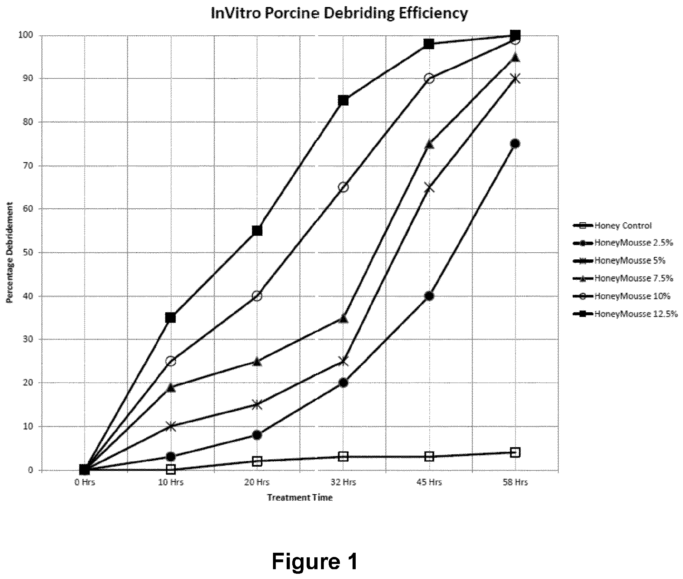

Figure 1 shows the debriding or desloughing efficiency of each of the above compositions, when applied to a sample of pig skin in vitro, at room temperature. Since the melanin pigment in pig skin is located primarily in the basal layer of the epidermis, detachment of the dark-coloured epidermis reveals the amber-coloured dermis beneath. Therefore, detachment of the epidermis may be easily visualized and was taken as a marker of successful debriding or desloughing of the epidermis from the dermis. Squares of pig skin, 2.5 cm2 in area, were treated with each of formulations (A) to (E) of Table 1, according to the following protocol, repeated every 12 hours: (1) wash with water, (2) photograph and quantify debrided area and (3) reapply 1.5 g of formulation to each square of pig skin (i.e. approximately 0.24 g per 1 cm2). The area of the revealed dermis was quantified on a grid and calculated as a percentage of the total area of the sample, at each 12 hour application point.

As shown in Figure 1, there was an incremental increase in debriding or desloughing efficiency, dependent on sodium bicarbonate concentration, which reached a plateau at around 12.5% w/w sodium bicarbonate, in Formulation (E).

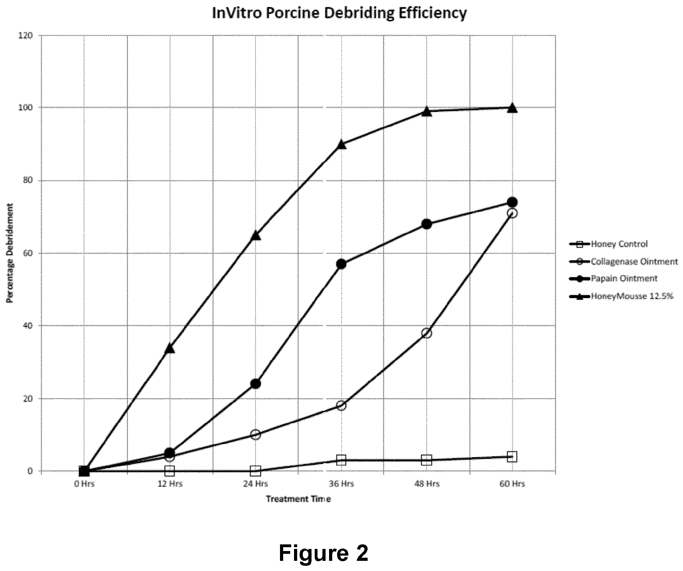

Formulation (E) was then compared with the debriding efficiency of commercially available debriding compositions, Santyl^{®} collagenase ointment (Smith & Nephew, Inc.) and Kovia^{®} papain-urea ointment (521,000 units papain activity per gram ointment; Stratus Pharmaceuticals Inc.). Debriding/desloughing efficiency was measured in vitro on pig skin, according to the same experimental protocol as described above. Figure 2 shows the results of the comparison of in vitro debriding efficiency of Formulation (E), containing 12.5 % w/w sodium bicarbonate, compared with commercially available debriding compositions, Santyl^{®} collagenase ointment (Smith & Nephew, Inc.) and Kovia^{®} papain-urea ointment (as above; Stratus Pharmaceuticals Inc.).

As shown in Figure 2, Formulation (E) comprising 12.5% w/w sodium bicarbonate provided faster and more effective debridement of the pig skin, compared to either Kovia^{®} papain-urea ointment or Santyl^{®} collagenase ointment.

### (iii) In Vivo Tests of Debriding/Desloughing Efficiency

### (a) Treatment of equine myiasis ulcer on donkey

A donkey with a large myiasis ulceration on lateral aspect of right front knee was treated with Formulation (E).

The ulceration wound was 8.6 cm wide by 9.4 cm high and presented with heavy encrustation of blood. The wound was cleansed with 3% hydrogen peroxide and irrigated with sterile saline on Day 0 and was treated to debride and heal by secondary intention. The study consisted of daily removal of the dressing and saline irrigation followed by application of the following at the given time periods.

Day 0: Pre-Debriding Ointment (as provided in U.S. Patent Application Publication No. 20130085461) was applied for 24 hours. Ambient temperature was 32-35°C. Dressed with non-stick dressing and wrapped with leg pad and held with cloth strap and Velcro^{®} closure.
Days 1-7: Kiwi fruit juice concentrate (containing naturally-occurring actinidin) plus formulation A (Leptospermum scoparium (Manuka) honey and 2.5% sodium bicarbonate, pH 6.35);
Days 8-29: Medical grade Manuka Leptospermum scoparium honey only, pH 3.63 (Links Medical LLC);
Days 30-68: Formulation E (Leptospermum scoparium honey and 12.5% sodium bicarbonate, pH 7.2)

The composition of the Pre-Debriding Ointment was as follows (also as provided in U.S. Patent Application Publication No. 20130085461):

| **Ingredient** | **Concentration (% W/W)** |
|---|---|
| Urea | 25.00 |
| Waxes and mineral oils | 10.00 |
| PEG-100 (Carbowax^{®}) | 6.00 |
| lidocaine hydrochloride | 4.00 |
| n-propanol | 4.00 |
| lanolin | 4.00 |
| cetyl alcohol | 3.00 |
| glucose (USP grade) | 2.00 |
| mandelic acid | 2.00 |

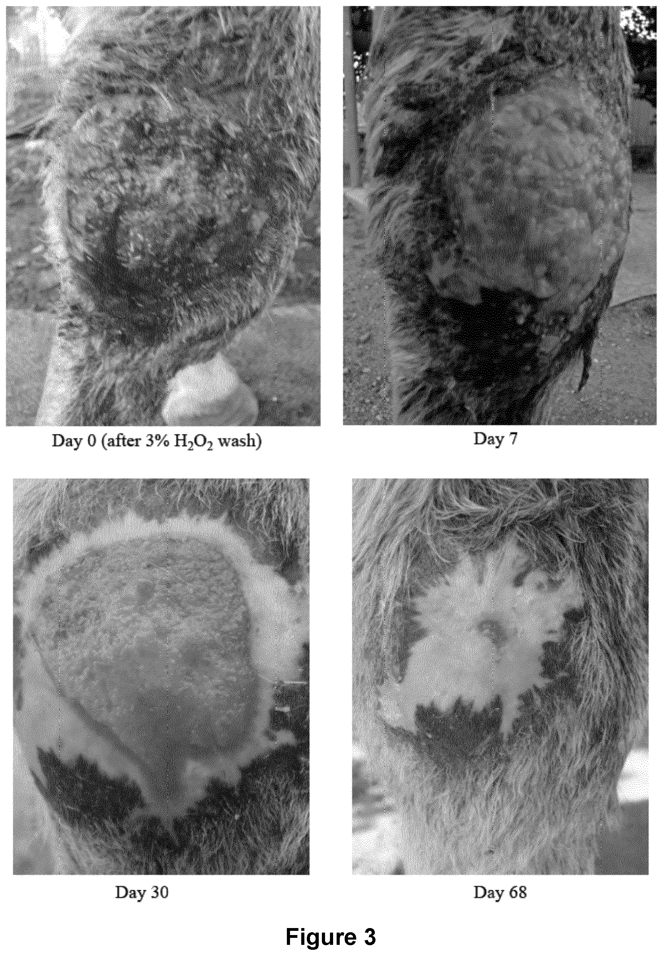

During debriding (days 1-7), treatment was daily. After debriding during granulation and remodeling of granulation tissue (days 8-29), treatment was every 3 to 4 days. During dermal and epidermal tissue construction and migration, to wound closure, the treatment composition was applied every 4 to 7 days. Photographs were taken during treatment; Figure 3 shows photographs taken on days 0, 7, 30 and 68 of the treatment. Observations at the end of each stage of treatment are as provided below.

Following the pre-debriding ointment treatment for 24 hours at 32-35°C on heavy eschar and necrotic tissue, the subsequent use of kiwi fruit juice enzyme (actinidin) in honey mousse was able to debride the wound in 4 days and promote rapid granulation. Debriding was continued with the treatment composition to day 7 to ensure complete cleansing. At day 7, the wound appeared to be vigorously granulating with good vascularity and has filled the wound deficit. Medical grade Manuka (Leptospermum scoparium) honey alone was used on days 8 to 28, and produced good granulation development and remodeling up to Day 30. At day 30, epithelial migration continued to close the wound. Granulation tissue is also continuing to remodel and vascularize and innervate.

Formulation (E) from Table 1 continued the healing with epidermal migration starting around day 30. Formation of new epithelium was about 75% complete at 49 days, about 90% complete at 63 days, and about 93% complete at 68 days. This rate of epithelialization appeared to be slightly delayed and indicates that the honey mousse (12.5% sodium bicarbonate) may be best suited for the debriding and granulation phase of wound healing.

### (b) Treatment of equine myiasis ulcer on donkey

A donkey with ankle wounds was presented for treatment. Myiasis ulcerations 2.5 cm x 1.5 cm were located on the anterior aspect of the front right ankle. The donkey had been plagued by fly bite irritated skin resulting in ulceration and erosion of epidermis and dermis. Left uncovered and untreated, this ulceration would enlarge rapidly as did the ulceration on his knee above.

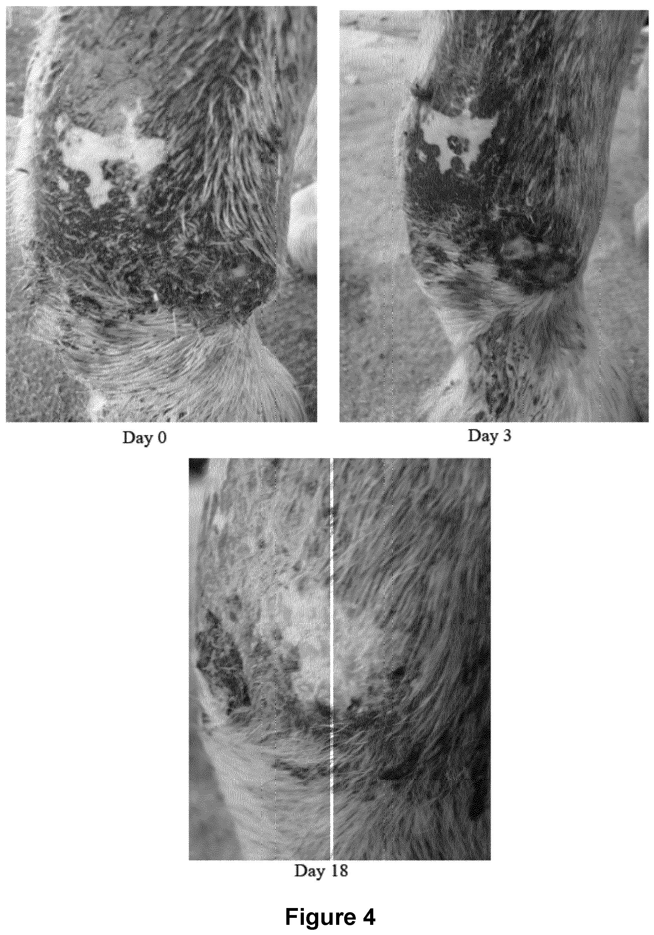

The affected area was treated with Formulation (A) and then Formulation (E), according to the following protocol. Photographs were taken during treatment; Figure 4 shows photographs taken on days 0, 3 and 18 of the treatment. Observations at the end of each stage of treatment are as provided below.

Day 0: Pre-Debriding Ointment (as noted above in Example 1(iii)(a); as provided in U.S. Patent Application Publication No. 2013/0085461) was applied and left in place for 24 hours. Ambient temperature was 35°C.

Day 1-3: Kiwi fruit juice with actinidin enzyme, 40 units/g, was added to formulation (A) (Manuka Leptospermum scoparium honey and 2.5% w/w sodium bicarbonate), and an amount sufficient to cover wound was applied to the wound site. For 3 days, the wound was washed with saline and the non-stick dressing was changed every 24 hours. The primary dressing was with cotton wrap with Velcro^{®} closure. Debriding was considered complete on day 3. The development of new granulation tissue was observed on day 3.

Day 4-18: The wound site was treated with Formulation (E) (Manuka Leptospermum scoparium honey and 12.5% sodium bicarbonate). Honey mousse left in place for 3-4 days at a time. On day 18, the wound was observed as being fully closed.

### (c) Treatment of equine myiasis ulcer on horse

A horse with a large girth (chest) wound from myiasis (fly bite) ulcerations measuring 10 cm x 9.5 cm x 1.2 cm deep, was presented for treatment. The wound site was surgically debrided and treated with Formulation (E) (Manuka honey and 12.5% w/w sodium bicarbonate) until closure according to the following protocol.

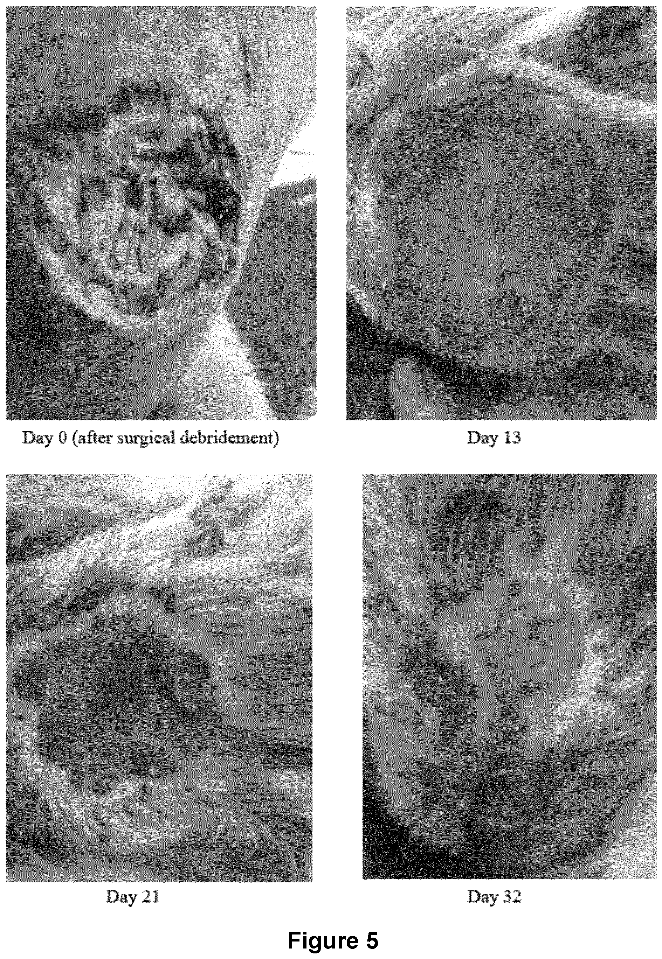

The horse was given appropriate sedatives and was then positioned on its back. The chest wound, found just distal to the front legs, was cleansed with betadine, and the area surrounding the wound site was shaved. The necrotic tissue mass was surgically excised down into the grey fibrous connective tissue over the sternum, approximately 1.5cm deep. The wound site was irrigated with sterile saline and Formulation (E) was applied to non-stick pads and placed on the wound. A secondary dressing of a disposable diaper was used to further secure Formulation (E) in place. On subsequent dressing changes, the wound was irrigated, photographed and Formulation (E) reapplied on non-stick dressings held in place with a custom-made sling created by the owner which kept the dressing secure for 3-7 days at a time. Photographs were taken during treatment; Figure 5 shows photographs taken on days 0, 16, 21 and 32 of the treatment. Observations at the end of each stage of treatment are as provided below.

Formulation (E) was effective for debriding the wound margin tissue that became necrotic after the surgical excision, and was a strong stimulator of granulation and remodeling. By day 16, the wound had finished granulation and was remodeling well out to the edges. By day 21, epithelial migration had started and it was decided to continue application of Formulation (E) to wound closure, in order to get a second assessment of its effect during this phase. The wound closed on day 32, well within normal time frame. The new epithelium appeared soft and loosely organized, as observed during the treatment described in Example 1(iii)(a).

### (d) Treatment of surgical excision wound on human male patient

A human patient, male, age 69 years, in good general health, was presented with a skin pathology appearing to be a classic keratoacanthoma on his right dorsal forearm. The keratoacanthoma lesion was approximately 1 cm in diameter with a central crater, raised, with no erythema. A shave biopsy was taken of the raised portion, approximately 1.4 x 1.3 x 0.2 cm in size, with an eccentric 0.9 x 0.7 cm white and brown lesion. At excision, the capillary bleeding was managed with electrocautery. The shave biopsy specimen was sent out for pathology analysis.

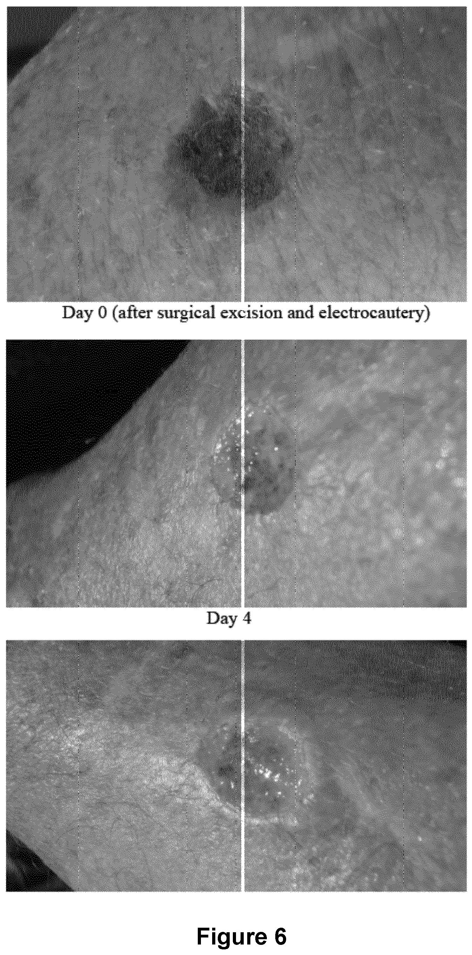

The surgical wound was closed by secondary intention and treated daily with Formulation (E) starting the day after the excision. The wound cavity was filled with Formulation (E) and covered with an island type secondary bandage. At each daily dressing change, the wound was irrigated and re-dressed with Formulation (E). The treatment was halted on day 7, when the pathology analysis found that the biopsy as comprising squamous cell carcinoma, invasive and well differentiated, present at the deep margin. Photographs were taken during treatment; Figure 6 shows the photographs taken on days 0, 5 and 7 of the treatment. Observations at the end of each stage of treatment are as provided below.

On day 0, shave biopsy and electrocautery produced a surgical excision wound site. The wound site was dressed with triple antibiotic ointment by the attending physician and covered with non-stick pad with paper tape. On day 4, formation of granulation was proceeding and the wound site appeared to be approximately half filled. By day 6, the granulation phase had completely filled the wound site, and the inflammatory response has subsided. By day 7, it was observed that rapid debriding of cautery point necrotic tissue had occurred, and granulation tissue had filled the wound site, with indications of angiogenesis.

The patient stated that there was no discomfort with the use of Formulation (E) from a very slight tingling sensation starting about 10 minutes after application and lasting about 10 minutes during the first two applications. This minimal sensation compared favorably against the stinging and burning sensations experienced by users of commercially-available enzymatic debriding compositions such as papain-based debriding ointments.

### Example 2: System for Wound Treatment

As an example, Formulation (E) of Example 1 was diluted with an isotonic diluent buffer solution as follows:

**Table 2 (a): Composition of Isotonic Diluent Buffer Solution**

| **Buffer Solution Ingredient** | **Concentration** |
|---|---|
| Ascorbic acid | 0.015 M |
| Sodium bicarbonate (NaHCO₃) | 0.015 M |
| Sodium chloride (NaCl) | 0.12 M |

Undiluted Formulation (E) has an approximate osmolarity (also referred to as osmotic concentration) of 14.5 times greater than human blood. Formulation (E) of Example 1 was diluted with the isotonic diluent solution of Table 2(a), at the following dilution factors provided in Table 2(b) below, with the corresponding resultant osmolarity compared to human blood.

**Table 2 (b): Dilution Factors**

| **Dilution factor (Formulation (E) : Diluent solution)** | **Resultant hyperosmolarity of diluted formulation compared to human blood** |
|---|---|
| 1 : 2 | 7.2 x |
| 1 : 5 | 3.8 x |
| 1 : 10 | 2.3 x |
| 1 : 20 | 1.46 x |
| 1 : 30 | 1.15 x |

As noted above, the hyperosmolarity of the resultant solution was the osmotic concentration of the resultant solution when compared to human blood. For example, a solution diluted 1:2 resulted in a solution that had an osmotic concentration 7.2 x the osmotic concentration of human blood.

To each of these diluted solutions of Table 2(b), the following compounds were added to provide the final finished wound irrigation solutions.

**Table 2(c): Additives to diluted solution**

| **Additive** | **Final concentration in solution** |
|---|---|
| Sodium lauryl sulfate | 10 mM |
| polyaminopropyl biguanide | 0.00003% w/w |
| Disodium EDTA | 0.025% w/w |

The resultant diluted compositions were used as wound irrigation solutions, i.e. to irrigate the wound before applying a formulation of Example 1 (undiluted) for the first time to a wound site), or while replacing applications of the formulations of Example 1 to a wound site with an application of fresh formulation.

Various medical devices may be employed to apply the wound irrigation solution to the wound, including but not limited to, monofilament debriding sponges, pulsatile wound irrigation devices, mechanical spray irrigation devices (including spray and vacuum devices), and squeeze bottle dispensers.

### Example 3: Cosmetic Formulations

### (i) Sample Preparation

The following compositions, (a) and (b), for use as cosmetic formulations, were prepared.

### (a) Moisturizing lotion with 12% w/w sodium bicarbonate and 5% w/w honey

Each of phases A, B, C and D were blended to homogeneity and heated to the specified temperature range before final blending of all phases.

| **Phase** | **Ingredient** | **%, W/W** | **Weight (g)** |
|---|---|---|---|
| **Phase A** | Purified water | 48.0 | 480 |
| **(70-75°C )** | Carbopol^{®} 940 | 1.5 | 15 |
| | Mandelic acid | 1.5 | 15 |
| | Potassium sorbate | 0.2 | 2 |
| | Sodium benzoate | 0.2 | 2 |
| | Disodium EDTA | 0.1 | 1 |
| | Glycerin | 5.0 | 50 |
| | Urea | 10.0 | 100 |
| | Sodium bicarbonate | 12.0 | 120 |
| | | | |
| **Phase B** | Isopropyl myristate | 5.0 | 50 |
| **(75-80°C)** | Cetyl alcohol | 3.0 | 30 |
| | PEG-100 stearate | 2.0 | 20 |
| | | | |
| **Phase C** | Purified water | 5.0 | 50 |
| **( 45-50°C )** | Honey (Manuka, medical grade or regular) | 5.0 | 50 |
| | | | |
| **Phase D** | Benzyl alcohol | 0.5 | 5 |
| **( 35-40°C )** | Silk amino acids | 1.0 | 10 |
| | | | |
| | **TOTAL :** | **100.0** | **1000.000** |
| | **Standard pH : 8.50** | | |

### (b) Moisturizing lotion with 5% w/w sodium bicarbonate and 5% w/w honey

Each of phases A, B, C and D were blended to homogeneity and heated to the specified temperature range before final blending of all phases.

| **Phase** | **Ingredient** | **%, W/W** | **Weight (g)** |
|---|---|---|---|
| **Phase A** | Purified water | 54.5 | 545 |
| **(70-75°C)** | Carbopol^{®} 940 | 1.5 | 15 |
| | Mandelic acid | 2.0 | 20 |
| | Potassium sorbate | 0.2 | 2 |
| | Sodium benzoate | 0.2 | 2 |
| | Disodium EDTA | 0.1 | 1 |
| | Glycerin | 5.0 | 50 |
| | Urea | 10.0 | 100 |
| | Sodium bicarbonate | 5.0 | 50 |
| | | | |
| **Phase B** | Isopropyl Myristate | 5.0 | 50 |
| **(75-80°C)** | Cetyl alcohol | 3.0 | 30 |
| | PEG-100 Stearate | 2.0 | 20 |
| | | | |
| **Phase C** | Purified water | 5.0 | 50 |
| **(45-50°C)** | Honey (medical grade, Manuka or regular) | 5.0 | 50 |
| | | | |
| **Phase D** | Benzyl alcohol | 0.5 | 5 |
| **(35-40°C)** | Silk amino acids | 1.0 | 10 |
| | | | |
| | **TOTAL :** | **100.0** | **1000** |
| | **Standard pH : 7.57** | | |

### (ii) In Vivo Test

A female patient suffering from severe ichthyosis was a test subject. Previously, she had been using a urea and alpha hydroxy acid skin lotion (Extra Strength Body Lotion, Dermal Therapy Inc.) for several years, in order to manage the symptoms of ichthoysis. The patient applied approximately 2 g of the moisturizing lotion as provided in Example 2(i)(a) above, comprising 12% sodium bicarbonate and 5% honey, to an affected area on one arm, twice daily, for a period of two weeks. She observed a comparable improvement to her symptoms as with the urea-lactic acid cream.

Although the invention has been described with reference to certain specific embodiments, various modifications thereof will be apparent to those skilled in the art without departing from the purpose and scope of the invention as outlined in the claims appended hereto. Any examples provided herein are included solely for the purpose of illustrating the invention and are not intended to limit the invention in any way. Any drawings provided herein are solely for the purpose of illustrating various aspects of the invention and are not intended to be to limit the invention in any way..

### Example 4: Wound Dressing Incorporating Dry Components

This example serves to illustrate a wound dressing incorporating Manuka honey in the form of freeze dried granules, sodium bicarbonate powder and ascorbic acid crystals.

### Method

1) Saturate sodium bicarbonate in high fructose corn syrup at 40 °C and saturate the dressing. Dry with 8-ply rayon/polyester layer of dressing on non-stick conveyor belt with cellulose coated layer on top, using warm (40 °C) air until completely dry and binding the two layers together.
2) Blend ascorbic acid crystals and Manuka honey freeze dried granules (pH 3.6-4.5) in either a debriding or healing formulation proportion and coat with Eastman^{™} Cellulose Acetate Phthalate (CAP) using acetone:water (97%:3%) as the CAP solvent. Spread the wet slurry of honey granules and ascorbic acid crystals onto "cellulose tissue" dressing surface, and allow to evaporate to dryness under negative air flow. Sodium bicarbonate is less than 0.2% soluble in acetone, so almost none will be dissolved.
3) When dried, seal the dressing in a typical dressing sleeve and irradiate to sterilize.

More specifically, the method of this example involves the following. Micro-encapsulate freeze-dried Manuka honey granules blended with ascorbic acid crystals (pH3.6-4.5) in Cellulose Acetate Phthalate (CAP) and adhere the encapsulated mixture to a cellulose tissue layer of a gauze dressing. CAP is typically used for pharmaceutical encapsulation to produce an enteric coating that resists dissolution when in an acid environment, but dissolves when exposed to neutral to alkaline solution. Adhere sodium bicarbonate to the outer rayon/polyester layer of the gauze dressing. Since the wound fluid is absorbed by the dressing, the dissolving sodium bicarbonate solution (pH 7-8) will cause dissolution of the CAP coating and release the honey and ascorbic acid. The honey is extremely hygroscopic and will immediately absorb and rehydrate with wound fluid water to a honey consistency (about 14% water). The organic acids in the honey and ascorbic acid will react in the wound fluid water with dissolved sodium bicarbonate to release carbon dioxide which infuses the now fluid honey to create a foam.

Excess carbon dioxide is released from the non-occlusive gauze dressing. Occlusive adhesive border dressings should be avoided as they could inflate or even dislodge with carbon dioxide pressure.

The advantage of the sodium bicarbonate-fructose:glucose impregnation of the dressing is that it binds the sodium bicarbonate to the dressing (thereby preventing the powder from sifting through the dressing fabric) and it presents a saturated sodium bicarbonate solution in wound fluid to the necrotic tissue in the wound for maximum loosening, softening and detachment activity. The undissolved sodium bicarbonate in the dressing acts as a reserve to continue to dissolve over time just as it does in fluid honey model. The CAP coating of the hygroscopic honey granules and ascorbic acid causes same to be adhered to the dressing and prevents them from pre-maturely absorbing water and ensuring a longer shelf-life even with normal dressing enclosures. The CAP coating withstands prolonged contact with acidic environments (the acidic honey and ascorbic acid), then dissolves on contact with slightly acidic (pH 6.2) to neutral (pH 7-8) solutions.

Initially, the high fructose corn syrup/sodium bicarbonate gel produces negative osmotic pressure as it hydrates, drawing in even more wound fluid and atmospheric moisture. Then the sodium bicarbonate dissolved in the wound fluid (pH 7-8) dissolves the CAP coating and releases honey and ascorbic acid. The freeze dried honey granules then hydrate and increase the negative osmotic pressure incrementally. Because the sodium bicarbonate reaction with acid is driven by the evolving carbon dioxide gas, the equilibrium is forced strongly in the direction of continued reaction until all the available sodium bicarbonate in the expanding "wet" area of the dressing is used up. The mechanism is self-regulating based on available wound fluid. This mechanism will work for both the "Debriding and the Healing" formulations.

### Example 5: Negative Osmotic Pressure Dressing for Treating Necrotic Wounds

Description: This dressing, or medical device, referred to as MANUKA-NW ,is used to soften and facilitate removal of necrotic wound tissue using MANUKA honey (100% Leptospermum scoparium honey from New Zealand). MANUKA-NW is super-saturated with sodium bicarbonate for necrotic tissue detachment, and pH adjusted to neutral with Vitamin C to optimize autolytic debriding enzyme activity. MANUKA-NW Activator (sodium bicarbonate and Vitamin C ) is mixed with MANUKA honey at Point-Of-Use to generate carbon dioxide which foams the viscous honey. The high sugar level of MANUKA-NW provides Negative Osmotic Pressure to draw wound fluid and autolytic cleansing to the wound.

The following are some benefits of the activated MANUKA-NW:
1) Improves removal and application at dressing changes, since it is less sticky than honey.
2) Absorbs and/or neutralizes wound malodors with sodium bicarbonate.,
3) Expands into difficult-to-reach undermined or tunneled necrotic areas of the wound to provide moist wound environment.
4) Provides neutral pH needed for optimal autolytic debridement.
5) Provides a temporary CO₂ rich atmosphere over the wound to discourage growth of aerobic bacteria.
6) Manuka honey supersaturated with sodium bicarbonate rapidly loosens, softens and detaches necrotic epidermis and dermis tissue.

The Activated MANUKA NW is applied to the wound containing necrotic tissue and wound debris, covered with gauze (which may be supplied as part of the kit), then secured in place with a suitable secondary dressing or wrap.

The contents of the kit could preferably comprise:
1) 0.5oz. (14g) sterile 100% Manuka (Leptospermum scoparium) Honey in a flexible tube with foil seal under threaded cap.
2) Activator: 0.55g ascorbic acid and 2.35g sodium bicarbonate powder in sealed container.

### Example 6: Negative Osmotic Pressure Dressing for Healing Wounds

Description: This dressing, or medical device, referred to as MANUKA N.O.P.D. is primarily designed for healing wounds and ulcers using Manuka Honey (100% Leptospermum scoparium Honey from New Zealand) in a sterile container, activated at point-of-use with ascorbic acid (Vitamin C) and sodium bicarbonate powder to create an expanding, pH adjusted (6.2), carbon dioxide (CO2) infused, honey with Negative Osmotic Pressure. The activated MANUKA N.O.P.D. is applied to a 4 in. X 4 in. foam pad dressing (included), then secured in place with a suitable secondary dressing or wrap.

Activated Manuka honey's high sugar level provides Negative Osmotic Pressure that promotes continuous autolytic debridement and helps maintain a moist environment conducive to wound healing. Sodium bicarbonate rapidly absorbs and neutralizes wound malodors. The CO₂ infused consistency of the activated honey provides:

The benefits of this device include:
1) Improved application and removal;
2) Expansion into undermined or tunneled areas of the wound without trauma or discomfort to the patient; and
3) Temporary CO₂ rich atmosphere over the wound to discourages growth of aerobic bacteria.

This medical device would contain:
1) 0.5oz. (14g) sterile 100% Manuka (Leptospermum scoparium) Honey in a flexible tube with foil seal under threaded cap;
2) Activator: 1.8g ascorbic acid and 0.6g sodium bicarbonate powder in sealed container.

### Example 7: Use of Manuka Honey Freeze Dried Granules Instead of Fluid Honey in Wound Dressings

Freeze-dried Manuka Honey granules were microencapsulated and blended with ascorbic acid crystals (pH 3.6 - 4.5) in Cellulose Acetate Phthalate (CAP) and the encapsulated mixture was adhered to the cellulose tissue layer of a gauze dressing. CAP is typically used for pharmaceutical encapsulation to produce an enteric coating that resists dissolution when in an acid environment, but dissolves when exposed to neutral to alkaline solution.

Sodium bicarbonate was adhered to the outer rayon/polyester layer of the gauze dressing. As wound fluid is absorbed by the dressing, the dissolving sodium bicarbonate solution (pH 7-8) will cause dissolution of the CAP coating and reveal the honey and ascorbic acid. The honey is extremely hygroscopic and will absorb and rehydrate with wound fluid water to a honey consistency (about 14% water). The honey organic acids and ascorbic acid will react in the wound fluid water with dissolved sodium bicarbonate to release carbon dioxide which infuses the now fluid honey to create the foam of this present patent application.

Excess carbon dioxide is released from the non-occlusive gauze dressing. Occlusive adhesive border dressings should be avoided as they could inflate or even dislodge with carbon dioxide pressure.

Although the above mentioned proportions of honey granules to ascorbic acid are preferably suited for use in a wound healing or treating formulation, a similar formulation can be used for the other uses mentioned in the present description. For example, in the case of a debriding formulation, the amount of the sodium bicarbonate component would be preferably higher while the proportion of the ascorbic acid would be less.

### References

1. Irish, J., Blair, S., Carter D.A. "The Antibacterial Activity of Honey Derived from Australian Flora." PLoS ONE. 2011, 6(3): e18229.
2. Kwakman, P. H. S. et al. "Two Major Medicinal Honeys Have Different Mechanisms of Bactericidal Activity." PLoS ONE. 2011, 6(3): e17709.
3. Atrott, J., Henle, T. "Methylglyoxal in Manuka honey - correlation with antibacterial properties." Czech Journal of Food Sciences. 2009, 27: S163-S165.
4. Smith, A. M., Hunt, N.C., Shelton, R. M., Birdi, G., and Grover, L.M. "Alginate Hydrogel Has a Negative Impact on In Vitro Collagen Deposition by Fibroblasts." Biomacromolecules. 2012, 13(12): 4032-4038.
5. Brandi, C. et al. "The Role of Carbon Dioxide Therapy in the Treatment of Chronic Wounds". In Vivo. 2010, 24: 223-226.

## Claims

1. A dressing for use in topical therapeutic treatment comprising at least a first layer and a second layer, wherein:
- the first layer comprises honey; and,
- the second layer comprises a pharmaceutically acceptable carbonate (CO₃⁻²) and/or bicarbonate (HCO₃⁻¹) salt,
- **characterized in that**: the honey is in a dry, granular form; and the carbonate and/or bicarbonate salt is in dry, granular form.

2. The dressing for use according to claim 1, wherein one or both of the first and second layers further comprises at least one organic acid, preferably in dry, granular form.

3. The dressing for use according to claim 2, wherein the at least one organic acid is gluconic acid, lactic acid, acetic acid, butyric acid, citric acid, malic acid, pyroglutamic acid, succinic acid, ascorbic acid, glycolic acid, lactic acid, citric acid, mandelic acid, or any combination thereof.

4. The dressing for use according to any one of claims 1 to 3, wherein the pharmaceutically acceptable carbonate or bicarbonate salt is sodium carbonate (Na₂CO₃), potassium carbonate (K₂CO₃), ammonium carbonate ((NH₄)₂CO₃), sodium bicarbonate (NaHCO₃), potassium bicarbonate (KHCO₃), ammonium bicarbonate ((NH₄)HCO₃) or any combination thereof.

5. The dressing for use according to any one of claims 1 to 3, wherein the pharmaceutically acceptable salt is a bicarbonate salt, preferably sodium bicarbonate (NaHCO₃), potassium bicarbonate (KHCO₃), ammonium bicarbonate ((NH₄)HCO₃) or any combination thereof.

6. The dressing for use according to claim 1, wherein the honey granules are encapsulated in a dissolvable coating.

7. The dressing for use according to claim 1, wherein the honey granules are adhered to the first layer.

8. The dressing for use according to any one of claims 1 to 7, wherein at least one of the first and second layers further comprises at least one proteolytic enzyme, wherein the at least one proteolytic enzyme is papain, actinidin, bromelain, ficain, cucumisin, a bacteria-derived proteolytic enzyme, or any combination thereof.

9. The dressing for use according to any one of claims 1 to 8, wherein at least one of the first and second layers further comprises at least one preservative, such as methyl paraben, propyl paraben, butyl paraben, imidazolidinyl urea, tetradecyl-trimethyl ammonium bromide (Cetrimide^{®}), sodium benzoate, potassium sorbate, thymol or polyaminopropyl biguanide, or any combination thereof.

10. The dressing for use according to any one of claims 1 to 9, wherein the at least one bicarbonate salt is present at 2.5 to 20% w/w, preferably 5 to 15% w/w, more preferably 7.5 to 12.5% w/w, of said composition.

11. The dressing for use according to claim 2 or 3, wherein the at least one organic acid is present in a concentration of 0.2 to 10% w/w, preferably 1 to 5% w/w, of said composition.

12. The dressing for use according to any one of claims 1 to 11, wherein the dressing is adapted for use in desloughing, debriding, wound treating, and/or exfoliating applications.

13. The dressing for use according to any one of claims 1 to 12, wherein the dressing is contained in a sealed package.

14. The dressing for use according to any one of claims 1 to 13, wherein the dressing is a facial mask.

15. A dressing for use according to any one of claims 1 to 14 for use in a desloughing, debriding, wound treating, and/or exfoliating.

16. The dressing for use according to claim 15, wherein the dressing is topically applied to a site on a subject for the desloughing, debriding, wound treating, and/or exfoliating.

## Patentansprüche

1. Wundauflage zur Verwendung bei topischer therapeutischer Behandlung, umfassend mindestens eine erste Schicht und eine zweite Schicht, wobei:
- die erste Schicht Honig umfasst; und
- die zweite Schicht ein pharmazeutisch akzeptables Carbonat-(CO₃⁻²) - und/oder Bicarbonat- (HCO₃⁻¹) -Salz umfasst,
- **dadurch gekennzeichnet, dass**: der Honig in trockener, granularer Form vorliegt; und das Carbonat- und/oder Bicarbonat-Salz in trockener, granularer Form vorliegt.

2. Wundauflage zur Verwendung nach Anspruch 1, wobei eine oder beide der ersten und zweiten Schicht ferner mindestens eine organische Säure, vorzugsweise in trockener, granularer Form, umfassen.

3. Wundauflage zur Verwendung nach Anspruch 2, wobei es sich bei der mindestens einen organischen Säure um Gluconsäure, Milchsäure, Essigsäure, Buttersäure, Zitronensäure, Äpfelsäure, Pyroglutaminsäure, Bernsteinsäure, Ascorbinsäure, Glykolsäure, Milchsäure, Zitronensäure, Mandelsäure oder eine beliebige Kombination davon handelt.

4. Wundauflage zur Verwendung nach einem der Ansprüche 1 bis 3, wobei es sich bei dem pharmazeutisch akzeptablen Carbonat- oder Bicarbonat-Salz um Natriumcarbonat (Na₂CO₃), Kaliumcarbonat (K₂CO₃), Ammoniumcarbonat (NH₄)₂CO₃), Natriumbicarbonat (NaHCO₃), Kaliumbicarbonat (KHCO₃), Ammoniumbicarbonat ((NH₄)HCO₃) oder eine beliebige Kombination davon handelt.

5. Wundauflage zur Verwendung nach einem der Ansprüche 1 bis 3, wobei es sich bei dem pharmazeutisch akzeptablen Salz um Bicarbonatsalz, vorzugsweise um Natriumbicarbonat (NaHCO₃), Kaliumbicarbonat (KHCO₃), Ammoniumbicarbonat ((NH₄)HCO₃) oder eine beliebige Kombination davon handelt.

6. Wundauflage zur Verwendung nach Anspruch 1, wobei die Honiggranulate in einer auflösbaren Beschichtung verkapselt sind.

7. Wundauflage zur Verwendung nach Anspruch 1, wobei die Honiggranulate an der ersten Schicht angehaftet sind.

8. Wundauflage zur Verwendung nach einem der Ansprüche 1 bis 7, wobei mindestens eine der ersten und zweiten Schichten ferner mindestens ein proteolytisches Enzym umfasst, wobei es sich bei dem mindestens einen proteolytischen Enzym um Papain, Actinidin, Bromelain, Ficain, Cucumisin, ein aus Bakterien gewonnenes proteolytisches Enzym oder eine beliebige Kombination davon handelt.

9. Wundauflage zur Verwendung nach einem der Ansprüche 1 bis 8, wobei mindestens eine der ersten und zweiten Schicht ferner mindestens ein Konservierungsmittel wie Methylparaben, Propylparaben, Butylparaben, Imidazolidinylharnstoff, Tetradecyltrimethylammoniumbromid (Cetrimid^{®}), Natriumbenzoat, Kaliumsorbat, Thymol oder Polyaminopropylbiguanid oder eine beliebige Kombination davon umfasst.

10. Wundauflage zur Verwendung nach einem der Ansprüche 1 bis 9, wobei das mindestens eine Bicarbonat-Salz in einer Menge von 2,5 bis 20 Gew.-%, vorzugsweise 5 bis 15 Gew.-%, besonders bevorzugt 7,5 bis 12,5 Gew.-% der Zusammensetzung vorliegt.

11. Wundauflage zur Verwendung nach Anspruch 2 oder 3, wobei die mindestens eine organische Säure in einer Konzentration von 0,2 bis 10 Gew.-%, vorzugsweise 1 bis 5 Gew.-%, der Zusammensetzung vorliegt.

12. Wundauflage zur Verwendung nach einem der Ansprüche 1 bis 11, wobei die Wundauflage zur Verwendung bei der Belagentfernung, dem Debridement, der Wundbehandlung und/oder der Exfoliation angepasst ist.

13. Wundauflage zur Verwendung nach einem der Ansprüche 1 bis 12, wobei die Wundauflage in einer versiegelten Verpackung enthalten ist.

14. Wundauflage zur Verwendung nach einem der Ansprüche 1 bis 13, wobei es sich bei der Wundauflage um eine Gesichtsmaske handelt.

15. Wundauflage zur Verwendung nach einem der Ansprüche 1 bis 14 zur Verwendung bei der Belagentfernung, dem Debridement, der Wundbehandlung und/oder der Exfoliation.

16. Wundauflage zur Verwendung nach Anspruch 15, wobei die Wundauflage für die Belagentfernung, das Debridement, die Wundbehandlung und/oder die Exfoliation topisch auf eine Stelle eines Subjekts aufgebracht wird.

## Revendications

1. Pansement pour une utilisation dans un traitement thérapeutique topique comprenant au moins une première couche et une seconde couche, dans lequel :
- la première couche comprend du miel ; et,
- la seconde couche comprend un sel de carbonate (CO₃⁻²) et/ou de bicarbonate (HCO₃⁻¹) pharmaceutiquement acceptable,
- **caractérisé en ce que** : le miel se présente sous forme granulaire sèche ; et le sel de carbonate et/ou de bicarbonate se présente sous forme granulaire sèche.

2. Pansement pour une utilisation selon la revendication 1, dans lequel l'une ou les deux des première et seconde couches comprennent également au moins un acide organique, de préférence sous forme sèche et granulaire.

3. Pansement pour une utilisation selon la revendication 2, dans lequel l'au moins un acide organique est l'acide gluconique, l'acide lactique, l'acide acétique, l'acide butyrique, l'acide citrique, l'acide malique, l'acide pyroglutamique, l'acide succinique, l'acide ascorbique, l'acide glycolique, l'acide lactique, l'acide citrique, l'acide mandélique, ou une quelconque combinaison de ceux-ci.

4. Pansement pour une utilisation selon l'une quelconque des revendications 1 à 3, dans lequel le sel de carbonate ou de bicarbonate pharmaceutiquement acceptable est le carbonate de sodium (Na₂CO₃), le carbonate de potassium (K₂CO₃), le carbonate d'ammonium ((NH₄)₂CO₃), le bicarbonate de sodium (NaHCO₃), le bicarbonate de potassium (KHCO₃), le bicarbonate d'ammonium ((NH₄)HCO₃) ou une quelconque combinaison de ceux-ci.

5. Pansement pour une utilisation selon l'une quelconque des revendications 1 à 3, dans lequel le sel pharmaceutiquement acceptable est un sel de bicarbonate, de préférence le bicarbonate de sodium (NaHCO₃), le bicarbonate de potassium (KHCO₃), le bicarbonate d'ammonium ((NH₄)HCO₃) ou une quelconque combinaison de ceux-ci.

6. Pansement pour une utilisation selon la revendication 1, dans lequel les granules de miel sont encapsulés dans un enrobage soluble.

7. Pansement pour une utilisation selon la revendication 1, dans lequel les granules de miel sont collés à la première couche.

8. Pansement pour une utilisation selon l'une quelconque des revendications 1 à 7, dans lequel au moins l'une des première et seconde couches comprend également au moins une enzyme protéolytique, dans lequel l'au moins une enzyme protéolytique est la papaïne, l'actinidine, la bromélaïne, la ficaïne, la cucumisine, une enzyme protéolytique dérivée de bactéries, ou une quelconque combinaison de celles-ci.

9. Pansement pour une utilisation selon l'une quelconque des revendications 1 à 8, dans lequel au moins une des première et seconde couches comprend également au moins un agent de conservation, tel que le méthylparabène, le propylparabène, le butylparabène, l'imidazolidinylurée, le bromure de tétradécyltriméthylammonium (Cetrimide^{®}), le benzoate de sodium, le sorbate de potassium, le thymol ou le polyaminopropyl biguanide, ou une quelconque combinaison de ceux-ci.

10. Pansement pour une utilisation selon l'une quelconque des revendications 1 à 9, dans lequel au moins un sel de bicarbonate est présent à raison de 2,5 à 20 % p/p, de préférence 5 à 15 % p/p, de manière davantage préférée 7,5 à 12,5 % p/p, de ladite composition.

11. Pansement pour une utilisation selon la revendication 2 ou 3, dans lequel au moins un acide organique est présent à raison d'une concentration de 0,2 à 10 % p/p, de préférence de 1 à 5 % p/p, de ladite composition.

12. Pansement pour une utilisation selon l'une quelconque des revendications 1 à 11, dans lequel le pansement est conçu pour une utilisation dans des applications de détersion, de débridement, de traitement des plaies et/ou d'exfoliation.

13. Pansement pour une utilisation selon l'une quelconque des revendications 1 à 12, dans lequel le pansement est contenu dans un emballage scellé.

14. Pansement pour une utilisation selon l'une quelconque des revendications 1 à 13, dans lequel le pansement est un masque pour le visage.

15. Pansement pour une utilisation selon l'une quelconque des revendications 1 à 14, destiné à être utilisé pour la détersion, le débridement, le traitement des plaies et/ou l'exfoliation.

16. Pansement pour une utilisation selon la revendication 15, dans lequel le pansement est appliqué localement sur une zone d'un sujet pour la détersion, le débridement, le traitement des plaies et/ou l'exfoliation.
